# EUROPEAN PATENT APPLICATION

(11) **EP 4 339 894 A1**
(43) Date of publication of application: **20.03.2024**
(21) Application number: 23195382.9
(22) Date of filing: 05.09.2023
(51) Int. Cl.: G06T 11/00

(54) **X-RAY CT APPARATUS, DATA PROCESSING METHOD, AND STORAGE MEDIUM**

(30) Priority: 06.09.2022 JP 2022141201
(71) Applicant: Canon Medical Systems Corporation, Tochigi 324-0036 (JP)
(72) Inventor: MIYAZAKI, Hiroaki, Otawara-shi, 324-0036 (JP); SAITO, Yasuo, Otawara-shi, 324-0036 (JP)
(74) Representative: Marks & Clerk LLP

(57) **Abstract**

An X-ray CT apparatus (1) of an embodiment is provided with an acquisition unit (441) and a restoration unit (445). The acquisition unit (441) acquires first data in a first mode without coincidence counting correction. The restoration unit (445) restores, from the first data, second data having a higher spatial resolution than the first data.

## Description

### FIELD

Embodiments described herein and the drawings relate generally to an X-ray CT apparatus, a data processing method, and a storage medium.

### BACKGROUND

A photon-counting type X-ray detector used in an X-ray computed tomography (CT) apparatus measures the energy of each incident X-ray photon and assigns counts into an energy-bin by counting the number of photons. Here, the energy-bin means a range of X-ray spectra separated by a predetermined energy band. An X-ray CT apparatus using the photon-counting type X-ray detector can generate projection data for each energy band based on the counts assigned to each energy-bin and perform image reconstruction in each case of the projection data.

For example, direct conversion type detectors using semiconductor detection elements are known as the X-ray detector described above. In a direct conversion type detector, for example, electrodes are provided on a side facing semiconductor detection elements, such as cadmium telluride (CdTe) or cadmium zinc telluride (CdZnTe), to generate an electric field by the application of a bias voltage. In the direct conversion type detector, when X-rays are absorbed by the semiconductor detection element, electron-hole pairs are generated, electron transfer toward the anode side occurs, and the holes move toward the cathode side, resulting in a signal output.

In the direct conversion type detector, for example, a series of electrodes is formed on the cathode side, a plurality of electrodes are formed on the anode side, and individual charges based on electrons detected at individual electrodes on the anode side are read out; thereby, a signal of each pixel corresponding to each electrode on the anode side is output. In such a direct conversion type detector, when electrons are incident on the boundary between adjacent electrodes at the electrodes on the anode side, charge sharing, which causes some of the electrons to be detected at the electrodes on one side and causes the other electrons to be detected at the electrodes on the other side, may occur. When this charge sharing occurs, a charge read out from each of the two adjacent electrodes is a divided charge based on a single X-ray photon, resulting in reduced positional resolution and energy resolution.

In order to compensate for such charge sharing, an X-ray CT apparatus can execute charge sharing correction with coincidence counting values read out from two adjacent electrodes at the same time being used as output values from any of the electrodes, or a single-event-mode (SEM) to exclude coincidence counting values when the coincidence counting is observed.

### SUMMARY OF INVENTION

An X-ray CT apparatus provided in an aspect of the present embodiment includes an acquisition unit configured to acquire first data in a first mode without coincidence counting correction; and a restoration unit configured to restore second data having a higher spatial resolution than the first data by inputting the first data to a model.

The restoration unit may restore the second data by inputting the first data to a trained model.

The restoration unit may restore the second data from the first data by using an analytical model that analyzes a mutual relationship between the first data and the second data, or a function.

The second data may have approximately the same spatial resolution as that of data acquired in a second mode with coincidence counting correction.

The model may be trained based on third data acquired in the first mode and fourth data acquired in the second mode.

The third data and the fourth data may be individually acquired under a scanning condition in which counts are approximately the same as each other.

The fourth data may be acquired under a condition in which the amount of rays per unit time is higher than that of the third data.

The fourth data may be acquired under a condition in which a scan speed is slower than the third data.

The first mode may be a mode of double counting data between adjacent anode pixels.

The second mode may be a mode of discarding double-counted data between adjacent anode pixels.

The first data and the second data may be projection data, CT image data obtained by performing reconstruction processing on projection data, or image data obtained by performing image processing on CT image data.

A data processing method provided in an aspect of the present embodiment includes acquiring first data in a first mode without coincidence counting correction; and restoring second data having a higher spatial resolution than the first data by inputting the first data to a model.

A computer readable medium provided in an aspect of the present embodiment includes instructions that cause a computer to execute acquiring first data in a first mode without coincidence counting correction; and restoring second data having a higher spatial resolution than the first data by inputting the first data to a model.

### BRIEF DESCRIPTION OF THE DRAWINGS

FIG. 1 is a block diagram illustrating an example of the configuration of an X-ray CT apparatus according to a first embodiment;
FIG. 2A is a diagram illustrating an example of a circuit in an X-ray detector according to the first embodiment;
FIG. 2B is a diagram schematically illustrating a configuration of the X-ray detector according to the first embodiment;
FIG. 3 is a diagram illustrating an example of charge transfer in the X-ray detector according to the first embodiment;
FIG. 4 is a diagram illustrating examples of detection signals of the X-ray detector according to the first embodiment;
FIG. 5A is a diagram illustrating data collection by RCM according to the first embodiment;
FIG. 5B is a diagram illustrating data collection by SEM according to the first embodiment;
FIG. 6A is a diagram illustrating an example of a trained model construction according to the first embodiment;
FIG. 6B is a diagram illustrating an example of a trained model according to the first embodiment; and
FIG. 7 is a flowchart illustrating the processing procedure of the X-ray CT apparatus according to the first embodiment.

### DETAILED DESCRIPTION

Hereinbelow, an X-ray CT apparatus, a data processing method, and a data processing program according to an embodiment are described with reference to the accompanying drawings. Here, the X-ray CT apparatus, the data processing method, and the data processing program according to the present application are not limited to embodiments illustrated below.

The X-ray CT apparatus described in the following embodiments is an apparatus that can execute photon counting CT. An X-ray detector described in the following embodiments is a direct conversion type detector that directly converts X-ray photons into charges proportional to the energy thereof.

### First Embodiment

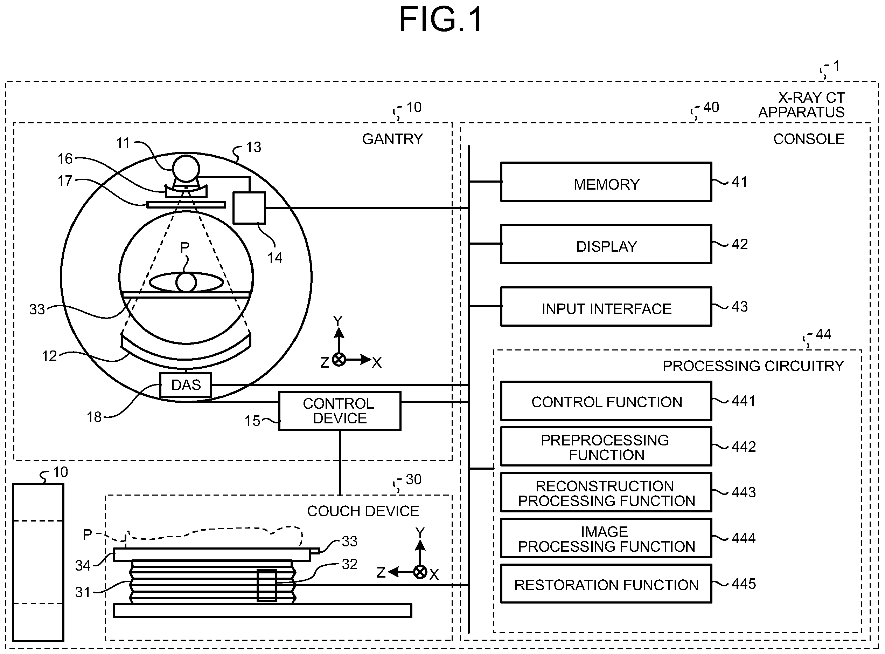

FIG. 1 is a diagram illustrating an example of the configuration of an X-ray CT apparatus 1 according to a first embodiment. As illustrated in FIG. 1, the X-ray CT apparatus 1 according to the first embodiment includes a gantry 10, a couch device 30, and a console 40.

Here, in FIG. 1, in the non-tilted state, a rotation axis of a rotating frame 13 or the longitudinal direction of a couchtop 33 of the couch device 30 is the Z-axis direction. An axis direction that is orthogonal to the Z-axis direction and horizontal to the plane of a floor is the X-axis direction. An axis direction that is orthogonal to the Z-axis direction and vertical to the plane of the floor is the Y-axis direction. FIG. 1 is a diagram for the purpose of illustrating the gantry 10 drawn in a plurality of directions and illustrating a case in which the X-ray CT apparatus 1 has one gantry 10.

The gantry 10 includes an X-ray tube 11, an X-ray detector 12, a rotating frame 13, an X-ray high voltage device 14, a control device 15, a wedge 16, a collimator 17, and a data acquisition system (DAS) 18.

The X-ray tube 11 is a vacuum tube with a cathode (filament) that generates thermal electrons and an anode (target) that generates X-rays upon impact of the thermal electrons. The X-ray high voltage device 14 applies high voltage to cause emission of thermal electrons from the cathode toward the anode; thereby, the X-ray tube 11 generates X-rays that are emitted to a subject P. For example, the X-ray tube 11 is an X-ray tube of a rotary anode type, which generates X-rays by emission of thermal electrons onto a rotary anode.

The rotating frame 13 is an annular frame that supports the X-ray tube 11 and the X-ray detector 12 facing each other and rotates the X-ray tube 11 and the X-ray detector 12 by the control device 15. For example, the rotating frame 13 is a cast made of aluminum as a material. The rotating frame 13 can further support the X-ray high voltage device 14, the wedge 16, the collimator 17, the DAS 18, and other components, in addition to the X-ray tube 11 and the X-ray detector 12. Furthermore, the rotating frame 13 can further support various configurations not illustrated in FIG. 1.

The wedge 16 is a filter that adjusts the amount of X-rays emitted from the X-ray tube 11. Specifically, the wedge 16 is a filter that transmits and attenuates X-rays emitted from the X-ray tube 11 such that X-rays emitted from the X-ray tube 11 to the subject P are distributed in a predetermined manner. For example, the wedge 16 is a wedge filter or a bow-tie filter, which is a filter made of aluminum or other material processed to have a predetermined target angle and a predetermined thickness.

The collimator 17 is a lead plate or the like to narrow down the emission range of the X-rays that are transmitted through the wedge 16, and a slit is formed with a plurality of the lead plates or the like in combination. The collimator 17 may also be referred to as an X-ray diaphragm. FIG. 1 illustrates a case in which the wedge 16 is disposed between the X-ray tube 11 and the collimator 17, but the collimator 17 may also be disposed between the X-ray tube 11 and the wedge 16. In this case, the wedge 16 transmits and attenuates X-rays, which are emitted from the X-ray tube 11 and whose emission range is limited by the collimator 17.

The X-ray high voltage device 14 includes a high voltage generating device that has electrical circuits, such as an electrical transformer (transformer) and a rectifier, and that generates high voltage to be applied to the X-ray tube 11, and an X-ray control device that controls output voltage according to X-rays generated by the X-ray tube 11. The high voltage generating device may be a transformer system or an inverter system. The X-ray high voltage device 14 may be provided in the rotating frame 13 or provided in a fixed frame not illustrated.

The control device 15 includes a processing circuit with a central processing unit (CPU) or other processing circuits, and a driving mechanism such as a motor and an actuator. The control device 15 receives input signals from an input interface 43 and controls the operation of the gantry 10 and the couch device 30. For example, the control device 15 controls the rotation of the rotating frame 13, the tilt of the gantry 10, and the operation of the couch device 30 and the couchtop 33. In one example, the control device 15 rotates the rotating frame 13 around an axis parallel to the X-axis direction based on input information on an inclination angle (tilt angle) as a control to tilt the gantry 10. The control device 15 may be provided in the gantry 10 or provided in the console 40.

The X-ray detector 12 outputs a signal, which enables the measurement on an energy value of an X-ray photon for each time when the X-ray photon is incident thereon. The X-ray photon is, for example, an X-ray photon emitted from the X-ray tube 11 and transmitted through the subject P. The X-ray detector 12 has a plurality of detection elements that output one pulse of electrical signal (analog signal) for each incident of X-ray photons. Counting the number of electrical signals (pulses) enables counting of the number of X-ray photons incident on each detection element. In addition, it is possible to perform arithmetic processing on this signal to measure an energy value of the X-ray photon that causes the output of the signal.

The X-ray detector 12 includes detection elements and an application specific integrated circuit (ASIC) that is connected to the detection elements and counts the X-ray photons detected by the detection elements. The ASIC counts the number of the X-ray photons incident on the detection elements by discrimination of individual charges output by the detection elements. The ASIC also measures the energy of the counted X-ray photons by performing arithmetic processing based on the magnitude of the individual charges. The ASIC outputs the results of counting the X-ray photons as digital data to the DAS 18.

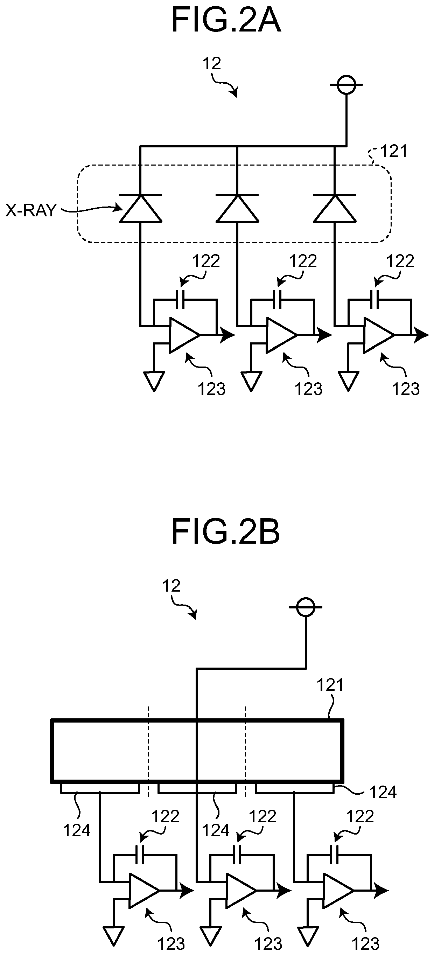

FIG. 2A is a diagram illustrating an example of a circuit in the X-ray detector 12 according to the first embodiment. Here, a circuit in which each configuration is arranged in one dimension is illustrated in FIG. 2A, but in practice, each configuration is arranged in two dimensions (matrix-like). For example, as illustrated in FIG. 2A, the X-ray detector 12 includes a detection element 121 that has a common electrode and a plurality of electrodes, in which the common electrode applies a high voltage (bias voltage) and is disposed on a surface onto which X-rays are incident, and the electrodes are disposed on a surface facing the surface onto which the X-rays are incident.

For example, the detection element 121 is a semiconductor detection element, such as cadmium telluride (CdTe) or cadmium zinc telluride (CdZnTe), with electrodes arranged thereon. That is, the X-ray detector 12 is a direct conversion type detector that converts incident X-ray photons directly into electrical signals.

Here, in the X-ray detector 12, an electric field is generated by the application of a bias voltage, the common electrode is used as a cathode electrode, and each of the electrodes is used as an anode electrode. That is, the X-ray detector 12 applies a bias voltage at the common electrode and reads out a charge generated by X-rays in the detection element 121 at each anode electrode. The cathode and anode are inverted according to the polarity of the applied voltage.

Each anode electrode is connected to the ASIC including a capacitor 122, an amplifier circuit 123, and other components. The capacitor 122 accumulates charges output from the anode electrodes, and the amplifier circuit 123 integrates the charges accumulated by the capacitor 122 and outputs the electrical quantity as a pulse signal. The ASIC also includes a wave-shaping circuit, a comparator circuit, a counter, and other components, calculates the counting result of X-ray photons based on the pulse signal, and outputs the calculated counting result as digital data to the DAS 18.

In the X-ray detector 12, each position corresponding to each anode electrode in the detection element 121 corresponds to a pixel. For example, in the X-ray detector 12, individual regions of the detection element 121 corresponding to the anode electrodes 124 respectively correspond to pixels, as illustrated in FIG. 2B. FIG. 2B is a diagram schematically illustrating a configuration of the X-ray detector 12 according to the first embodiment. Here, FIG. 2B is a schematic diagram corresponding to FIG. 2A.

The DAS 18 generates detection data based on the result of counting processing input from the X-ray detector 12. The detection data is, for example, a sinogram. The sinogram is data in which the counting processing results made incident on each of detection elements are arranged at each position of the X-ray tube 11. The sinogram is data in which the counting processing results are arranged in a two-dimensional Cartesian coordinate system with axis in a view direction and a channel direction. The DAS 18 generates a sinogram per column in a slice direction in the X-ray detector 12, for example. Here, the counting processing results are data in which the number of X-ray photons per energy-bin is assigned. For example, the DAS 18 counts photons (X-ray photons) derived from X-rays that are emitted from the X-ray tube 11 and transmitted through the subject P, and discriminates the energy of the counted X-ray photons to set a discriminated result as the result of the counting processing. The DAS 18 transmits the generated detection data to the console 40. The DAS 18 is implemented by, for example, a processor.

The data generated by the DAS 18 is transmitted via optical communication from a transmitter with light emitting diodes (LEDs), which is provided on the rotating frame 13, to a receiver with a photodiode, which is provided on a non-rotating portion of the gantry 10 (for example, fixed frame and the like, and not illustrated in FIG. 1), and is then forwarded to the console 40. Here, the non-rotating portion is, for example, a fixed frame that supports the rotating frame 13 in a rotatable manner. The method of transmitting data from the rotating frame 13 to the non-rotating portion of the gantry 10 is not limited to optical communication, and any non-contact data transmission method may be used, or a contact data transmission method may be employed.

The couch device 30 is a device over which the subject P who is an imaging target is placed and moved, and includes a base 31, a couch drive device 32, the couchtop 33, and a support frame 34. The base 31 is a housing that supports the support frame 34 in a vertically movable manner. The couch drive device 32 is a driving mechanism that moves the couchtop 33 on which the subject P is placed in a long axis direction of the couchtop 33, and includes a motor and an actuator. The couchtop 33 provided on the upper surface of the support frame 34 is a plate on which the subject P is placed. The couch drive device 32 may move, in addition to the couchtop 33, the support frame 34 along the long axis direction of the couchtop 33.

The console 40 includes a memory 41, a display 42, the input interface 43, and processing circuitry 44. The console 40 is described as a separate unit from the gantry 10, but the gantry 10 may include the console 40 or some of individual components of the console 40.

The memory 41 is implemented by, for example, a semiconductor memory such as a random access memory (RAM) or a flash memory, a hard disk, an optical disc, or the like. The memory 41 stores therein, for example, projection data, CT image data, trained model data, and other data. For example, the memory 41 also stores computer programs allowing circuits included in the X-ray CT apparatus 1 to achieve their functions. The memory 41 may also be implemented by a group of servers (cloud) connected to the X-ray CT apparatus 1 via a network.

The display 42 displays various types of information. For example, the display 42 displays various images generated by the processing circuitry 44 or displays a graphical user interface (GUI) for receiving various operations from an operator. For example, the display 42 is a liquid crystal display or a cathode ray tube (CRT) display. The display 42 may be a desktop type or may be configured as a tablet terminal or other device capable of wireless communication with a main body of the console 40.

The input interface 43 accepts various input operations from the operator, converts the accepted input operations into electrical signals, and outputs the electrical signals to the processing circuitry 44. For example, the input interface 43 accepts, from the operator, input operations such as reconstruction conditions when reconstructing CT image data and image processing conditions when generating post-processing images from CT image data.

For example, the input interface 43 can be implemented by a mouse, a keyboard, a trackball, a switch, a button, a joystick, a touch pad for input operation by a touch of the operation surface, a touch screen in which a display screen and a touch pad are integrated, a non-contact input circuit using an optical sensor, a voice input circuit, and other interfaces. The input interface 43 may be provided on the gantry 10. The input interface 43 may also be formed with a tablet terminal or other device capable of wireless communication with the main body of the console 40. The input interface 43 is not limited only to those with physical operating components such as a mouse or a keyboard. For example, an electrical signal processing circuit that receives electrical signals corresponding to input operations input from an external input device provided separately from the console 40 and outputs these electrical signals to the processing circuitry 44 is also an example of the input interface 43.

The processing circuitry 44 controls the operation of the entire X-ray CT apparatus 1. For example, the processing circuitry 44 executes a control function 441, a preprocessing function 442, a reconstruction processing function 443, an image processing function 444, and a restoration function 445. Here, for example, each processing function executed by the control function 441, the preprocessing function 442, the reconstruction processing function 443, the image processing function 444, and the restoration function 445, which are components of the processing circuitry 44 illustrated in FIG. 1, is recorded in the memory 41 in the form of a computer program executable by a computer. The processing circuitry 44 is, for example, a processor, which reads each computer program from the memory 41 and executes the computer program to execute the function corresponding to each computer program read out. In other words, the processing circuitry 44 with each computer program read out has each function in the processing circuitry 44 illustrated in FIG. 1.

The case in which each processing function of the control function 441, the preprocessing function 442, the reconstruction processing function 443, the image processing function 444, and the restoration function 445 is implemented by the single processing circuitry 44 is illustrated in FIG. 1, but the embodiment is not limited thereto. For example, the processing circuitry 44 may be composed of a plurality of independent processors in combination, and each processor may execute each computer program to execute each processing function. In addition, the processing circuitry 44 may be implemented with each processing function distributed or integrated into a single or multiple processing circuits as appropriate.

The control function 441 controls various processes based on input operations received from the operator via the input interface 43. Specifically, the control function 441 controls CT scans performed on the gantry 10. For example, the control function 441 controls a collection process of counting results in the gantry 10 by controlling the operation of the X-ray high voltage device 14, the X-ray detector 12, the control device 15, the DAS 18, and the couch drive device 32. The control function 441 also controls the display 42 to display various types of image data stored in the memory 41. The control function 441 is an example of an acquisition unit.

The preprocessing function 442 performs preprocessing such as logarithmic transformation processing, offset correction processing, sensitivity correction processing between channels, and beam hardening correction on detection data output from the DAS 18 to generate projection data.

The reconstruction processing function 443 performs reconstruction processing by using a filter-corrected inverse projection method, a successive approximation reconstruction method, or other method on projection data generated by the preprocessing function 442 or projection data restored by the restoration function 445, thereby generating CT image data. The reconstruction processing function 443 stores the reconstructed CT image data in the memory 41.

Here, the projection data generated based on the counting result obtained by photon counting CT contains information on energy of the X-rays attenuated by transmitting through the subject P. Thus, the reconstruction processing function 443 can reconstruct CT image data having a specific energy component, for example. The reconstruction processing function 443 can also reconstruct CT image data for each of a plurality of the energy components, for example.

The reconstruction processing function 443 can also, for example, assign a color tone to each pixel in the CT image data for each energy component according to an energy component and generate image data in which a plurality of pieces of the CT image data having the color tone assigned according to the energy component are superimposed. The reconstruction processing function 443 can also, for example, use the K-absorption edge specific to a substance to generate image data that enables the identification of the substance. Examples of other image data generated by the reconstruction processing function 443 include monochromatic X-ray image data, density image data, effective atomic number image data, and the like.

In order to reconstruct CT image data, projection data of 360°, circumference around the subject, is required, and also in the half-scan method, projection data of 180° plus fan angle is required. Any of the reconstruction methods can be applied to the present embodiment.

The image processing function 444 converts the CT image data generated by the reconstruction processing function 443 into image data such as tomogram of any cross-section or a three-dimensional image by rendering processing with known methods, based on an input operation received from the operator via the input interface 43. The image processing function 444 stores the converted image data in the memory 41.

The restoration function 445 restores data having a high S/N ratio and a high spatial resolution from data having a high signal-to-noise ratio (S/N ratio) and a low spatial resolution. The processing by the restoration function 445 will be described in detail later. The restoration function 445 is an example of a restoration unit.

The configuration of the X-ray CT apparatus 1 according to the first embodiment has been described above. Under such a configuration, the X-ray CT apparatus 1 enables the collection of images for easier observation in the image collection using the photon-counting type X-ray detector. Specifically, the X-ray CT apparatus 1 enables the collection of images with a high S/N ratio and a high spatial resolution in the image collection using the photon-counting type X-ray detector.

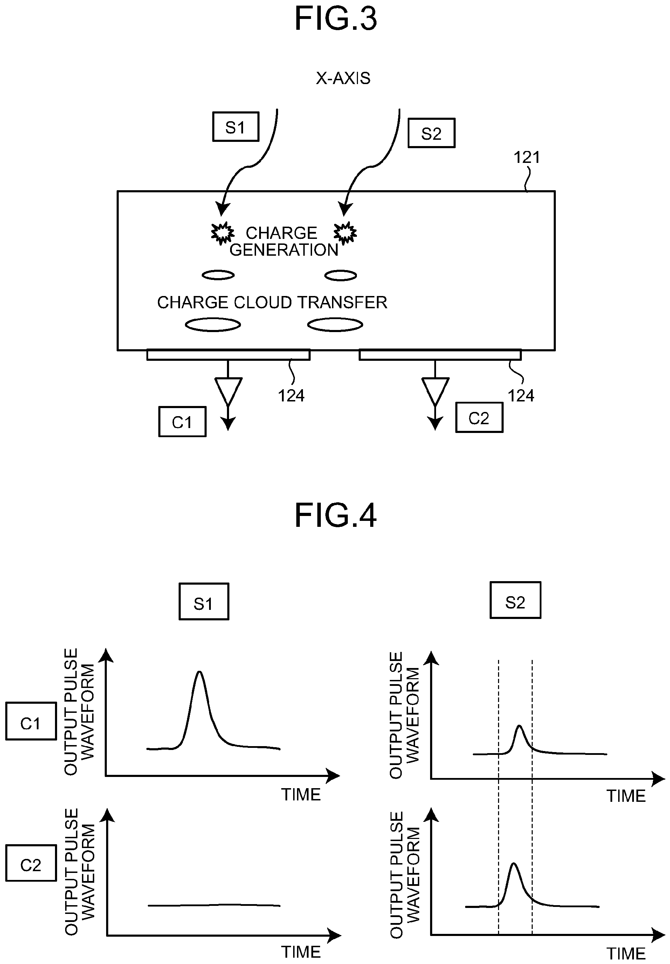

As described above, charge sharing may occur in the direct conversion type detector used as the photon-counting type X-ray detector when electrons are incident on the boundary between adjacent electrodes. FIG. 3 is a diagram illustrating an example of charge transfer in the X-ray detector 12 according to the first embodiment. In FIG. 3, "S1" and "S2" indicate incident X-rays, and "C1" and "C2" indicate readout circuits (such as capacitor 122 and amplifier circuit 123) disposed at respective positions.

As illustrated in FIG. 3, when "S1" and "S2" are incident on the detection element 121, electric charges are generated in the detection element 121. The generated charges move to the anode electrode 124 side by the bias voltage, but on the way, the charges gradually diffuse due to thermal motion and charge repulsion. Here, all the charges generated from "S1" and diffused are read out by "C1". On the other hand, the charges generated from "S2" and diffused are read out by each of "C1" and "C2".

FIG. 4 is a diagram illustrating examples of detection signals in the X-ray detector 12 according to the first embodiment. Examples of pulse signals detected by "C1" and "C2" for "S1" illustrated in FIG. 3 and examples of pulse signals detected by "C1" and "C2" for "S2" illustrated in FIG. 3 are illustrated in FIG. 4. For example, in the detection of "S1", pulse signals are output only by "C1" because all of the diffused charges are read out by "C1". On the other hand, in the detection of "S2", pulse signals are read by each of "C1" and "C2", and output by each of "C1" and "C2".

Here, each of the pulse signals has a wave height and an area corresponding to the energy amount of the X-ray photons. That is, a wave height value of each pulse signal is correlated with an energy value of the X-ray photons. Therefore, in the detection of "S1", a pulse signal having an accurate wave height value that reflects the energy of "S1" is output, resulting in a good energy resolution. On the other hand, in the detection of "S2", the charges are distributed to "C1" and "C2", resulting in inaccurate energy information (charge sharing). In the absence of a coincidence counting correction, no correction is applied to signals from a single x-ray incident on a detection element that are read-out by more than one electrode of the detection element.

In contrast, as charge sharing correction (coincidence counting correction), it is possible to execute the charge sharing correction with coincidence counting values read out from two readout circuits at the same time being used as output values from any of readout circuits, or the single-event-mode (SEM); however, images having sufficient quality may not be able to be collected. For example, for the charge sharing correction described above, a processing circuit is required to determine whether signals counted between the adjacent circuits at the same time are caused by the same X-ray, to add up the charges, and the like, and sufficient energy resolution may not be provided.

In the comparator counting commonly used today, the 120 keV range is divided into 6 bins with a width of 20 keV, and wave height values corresponding to the range are counted by 6 counters. As a result, the energy information (threshold value for dividing bins) is not dense, and it may be difficult to reconstruct the X-ray energy information accurately.

Hereinbelow, it will be described with an example to restore the energy information of "S2" from the two pulse signals corresponding to "S2" (the pulse signal output from "C1" and the pulse signal output from "C2") illustrated in FIG. 4. In each of bins divided into 6 bins, it is assumed that "bin 2" is 20 to 40 keV, "bin 3" is 40 to 60 keV, "bin 4" is 60 to 80 keV, and "bin 5" is 80 to 100 keV", the pulse signal of "S2" output from "C1" is "35 keV", and the pulse signal of "S2" output from "C2" is "55 keV".

In this case, the pulse signal output from "C1" is assigned to "bin 2" and the pulse signal output from "C2" is assigned to "bin 3". In this case, when the above-described charge sharing correction is performed (energy information is restored by bin addition), "bin 2 + bin 3" results in "bin 4", and the energy information of "S2" is "60 to 80 keV". In contrast, when the energy information is added, "35 keV + 55 keV = 90 keV", which is necessary to be actually used as "bin 5", resulting in the occurrence of an error.

In the above-described charge sharing correction, it is determined that charge sharing occurs even when charges based on different X-ray photons at adjacent anode electrodes are read out at the same time, resulting in the occurrence of false detection.

The SEM also uses the coincidence counting circuit to eliminate signals when the signals are read from adjacent electrodes during a certain time width. Therefore, the SEM may not be able to collect images having sufficient quality due to a low S/N ratio.

Therefore, in the present embodiment, data collected by an image collection mode with a relatively high S/N ratio and data collected by an image collection mode with a relatively high spatial resolution are used to generate data that complements the feature of each mode, thereby enabling the collection of images for easier observation. Specifically, the X-ray CT apparatus 1 uses a model based on the data collected by the image collection mode with a relatively high S/N ratio and the data collected by the image collection mode with a relatively high spatial resolution, thereby generating data having a high S/N ratio and a high spatial resolution.

For example, the X-ray CT apparatus 1 uses a trained model that has been trained using data collected by raw-count-mode (RCM), which outputs a signal from each anode electrode as a counting value and data collected by SEM to generate data having a high S/N ratio and a high spatial resolution.

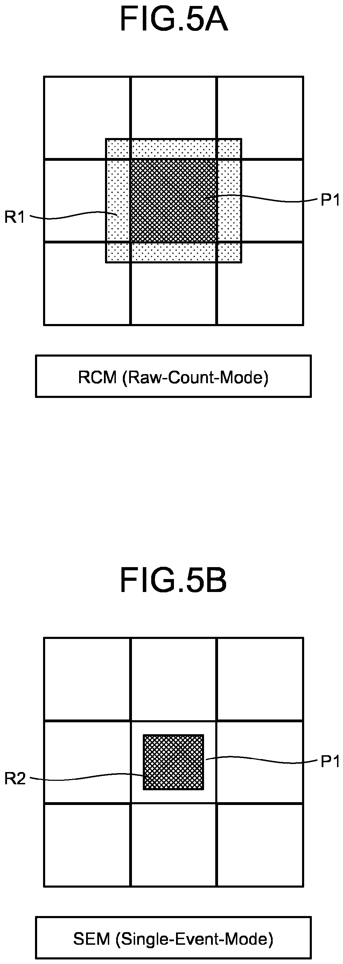

Here, the collection by RCM and the collection by SEM will be described with reference to FIGS. 5A and 5B. FIG. 5A is a diagram illustrating data collection by RCM according to the first embodiment; FIG. 5B is a diagram illustrating data collection by SEM according to the first embodiment. In FIGS. 5A and 5B, a part of the X-ray detector 12 (3 elements × 3 elements) with anode electrodes arranged in a two-dimensional matrix with respect to the detection element 121 is illustrated.

For example, in the collection by RCM, the charges generated by the incident X-rays are read out at each anode electrode. That is, in the RCM, pulse signals are output based on the charges read out at each anode electrode, without charge sharing correction. In other words, in the RCM, as illustrated in FIG. 5A, each of a pulse signal read out by a readout circuit corresponding to a center pixel P1 and a pulse signal read out by a readout circuit corresponding to an adjacent pixel region R1 (that is, each readout circuit corresponding to each of eight pixels surrounding the pixel P1) through charge sharing is output. Therefore, in the collection by the RCM, the counts are increased to cover the signals from the entire region corresponding to the pixels. Thus, the S/N ratio is increased, but the data is affected by charge sharing, resulting in data collection with the degraded spatial resolution and energy resolution.

In addition, for example, the collection by the SEM eliminates signals when the signals are read from adjacent anode electrodes at the same time. That is, as illustrated in FIG. 5B, in the SEM, even when X-rays are incident on the center pixel P1, only the X-rays incident on a region R2, which is narrower than the original region, are counted because the charges from the X-rays that are incident on positions near the edges of the pixel P1 are also read out at the adjacent anode electrodes, and the signals are thus eliminated. Therefore, in the collection by the SEM, the spatial resolution is high, and the energy resolution is also high because there is no impact from charge sharing; however, only signals from a narrow region are covered, so that data having a reduced S/N ratio is collected.

The X-ray CT apparatus 1 according to the present embodiment generates data having a high S/N ratio and a high spatial resolution by using the trained model trained with the data collected by the above-described RCM and the data collected by the above-described SEM. Here, the target data may be any of projection data, CT image data obtained by performing reconstruction processing on projection data, and image data obtained by performing image processing on CT image data, but an example of the case in which the projection data is used will be described below.

The control function 441 acquires first data in a first mode without coincidence counting correction. Specifically, the control function 441 controls the gantry 10 to collect the first data by the RCM on a subject. That is, the control function 441 controls data to be collected in a mode that double-counts data between adjacent anode pixels.

The restoration function 445 inputs the first data to the model to restore second data, which has a higher spatial resolution than that of the first data. Specifically, the restoration function 445 inputs projection data collected by the RCM on the subject to the trained model to restore projection data having a higher spatial resolution than that of the projection data collected by the RCM. More specifically, the restoration function 445 restores, from the projection data collected by the RCM, projection data having the same high S/N ratio as that of the projection data and having a higher spatial resolution than that of the projection data.

Here, the second data has approximately the same spatial resolution as the data acquired in a second mode, which performs coincidence counting correction. Specifically, the second data has a spatial resolution that is approximately the same as the data acquired by the SEM. That is, the second mode is a mode to discard double-counted data between adjacent anode pixels. In this way, the restoration function 445 restores, from the projection data collected by the RCM, projection data having a high S/N ratio, which is the same as that of the projection data, and projection data having a spatial resolution, which is approximately the same as that of the data collected by the SEM.

Here, prior to executing of the restoration processing on the projection data described above, the trained model is generated for an input of the projection data collected by the RCM in the present embodiment. Specifically, a trained model that has been trained based on third data acquired in the first mode (RCM) and fourth data acquired in the second mode (SEM) is generated.

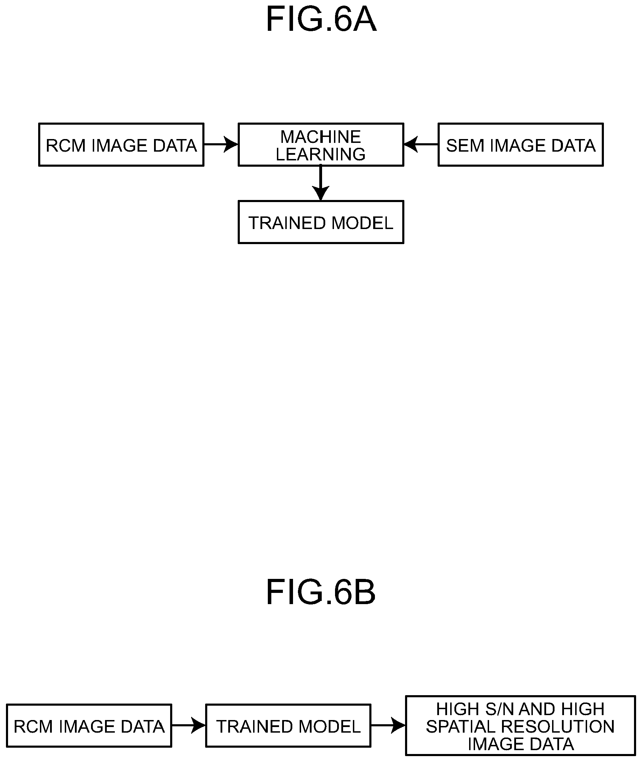

FIG. 6A is a diagram illustrating an example of a trained model construction according to the first embodiment; Here, the construction of the trained model illustrated in FIG. 6A may be executed by another information processing device (for example, workstation or the like) using the projection data in each mode collected by the X-ray CT apparatus 1, or may be executed by the X-ray CT apparatus 1. A device (information processing device, or X-ray CT apparatus 1) that constructs the trained model executes the following training function in a processing circuit included in the device.

For example, as illustrated in FIG. 6A, the training function generates the trained model by machine learning using a plurality of sets of the data set (training data) of the projection data collected by the RCM (RCM image data) and the projection data collected by the SEM (SEM image data).

Here, as described above, the projection data collected by the SEM has smaller counts and a lower S/N ratio as compared with the projection data collected by the RCM. Therefore, when projection data in each mode is collected under the same scan conditions and used as training data, the projection data having a low S/N ratio (much noise) is restored.

Therefore, in the present embodiment, when collecting the training data, the third data and the fourth data are individually acquired under a scan condition in which counts in the third data are approximately the same as those in the fourth data, with the same object used as a scan target. Here, in the present embodiment, when collecting the training data, for example, training data is collected on unknown data. For example, when collecting the third data by the RCM, the control function 441 collects data under the same scan condition as a scan condition under which data is actually collected from the subject, and when collecting the fourth data by the SEM, the control function 441 collects the data under a scan condition under which the counts are approximately the same as those in the third data. The control function 441 can switch between the collection by the RCM and the collection by the SEM by controlling the ASIC that the X-ray detector 12 has.

In one example, the control function 441 performs a control to acquire the fourth data (projection data collected by the SEM) with a condition in which the amount of rays per unit time is higher than that of the third data (projection data collected by the RCM). For example, the control function 441 also performs a control to acquire the fourth data (projection data collected by the SEM) with a condition in which the scan speed (rotation speed of the rotating frame 13) is slower than that of the third data (projection data collected by the RCM). The control function 441 controls the amount of rays and scan speed described above, alone or in combination, to execute a scan such that the counts in the fourth data is approximately the same as the counts in the third data.

The training function generates the trained model using the third data (projection data collected by the RCM) and the fourth data (projection data collected by the SEM) collected as described above as training data, and stores the generated trained model in a data server or the memory 41.

The restoration function 445 restores projection data by reading out the above-described trained model stored in the data server or the memory 41 and inputting the projection data collected by the RCM on the subject to the readout trained model. For example, as illustrated in FIG. 6B, the restoration function 445 inputs RCM image data acquired from the subject by the control function 441 to the trained model to acquire projection data having a high S/N ratio and a high spatial resolution. FIG. 6B is a diagram illustrating an example of the trained model according to the first embodiment.

The reconstruction processing function 443 performs reconstruction processing on the projection data restored by the restoration function 445 to generate CT image data. The image processing function 444 generates image data based on the CT image data reconstructed by the reconstruction processing function 443. The control function 441 controls the display 42 to display the image data generated by the image processing function 444.

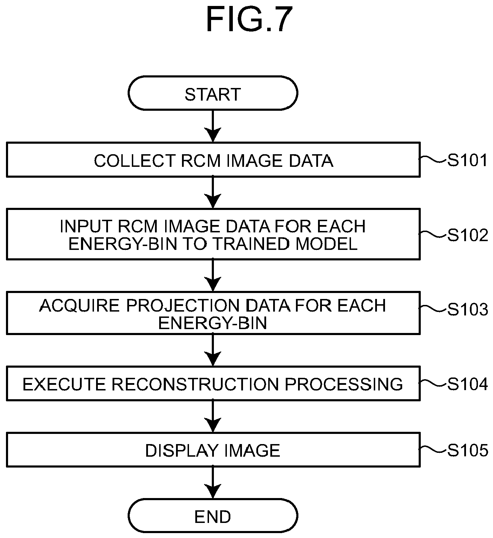

Next, the processing of the X-ray CT apparatus 1 according to the first embodiment will be described with reference to FIG. 7. FIG. 7 is a flowchart illustrating the processing procedure of the X-ray CT apparatus 1 according to the first embodiment.

For example, as illustrated in FIG. 7, in the present embodiment, the control function 441 collects data by the RCM to collect RCM image data in a scan on the subject (step S101). This processing is achieved by, for example, the processing circuitry 44 reading out a computer program corresponding to the control function 441 from the memory 41 and executing the computer program.

Subsequently, the restoration function 445 inputs the RCM image data (projection data) for each energy-bin collected by the control function 441 to the trained model (step S102) and acquires restored projection data for each energy-bin (step S103). This processing is achieved by, for example, the processing circuitry 44 reading out a computer program corresponding to the restoration function 445 from the memory 41 and executing the computer program.

Subsequently, the reconstruction processing function 443 executes reconstruction processing on the restored projection data for each energy-bin (step S104) to acquire CT image data. This processing is achieved by, for example, the processing circuitry 44 reading out a computer program corresponding to the reconstruction processing function 443 from the memory 41 and executing the computer program.

Subsequently, the control function 441 causes the display 42 to display an image generated based on the CT image data reconstructed by the reconstruction processing function 443 (step S105). This processing is achieved by, for example, the processing circuitry 44 reading out a computer program corresponding to the control function 441 from the memory 41 and executing the computer program.

As described above, according to the first embodiment, the control function 441 acquires the first data in the first mode without coincidence counting correction. The restoration function 445 inputs the first data to the model to restore the second data, which has a higher spatial resolution than that of the first data. Therefore, the X-ray CT apparatus 1 according to the first embodiment can restore data in which the counts has not been reduced to data having a high spatial resolution, which enables the collection of images for easier observation in the image collection by using the photon-counting type X-ray detector. For example, the X-ray CT apparatus 1 enables the collection of images having a high S/N ratio, a high spatial resolution, and a high energy resolution from the data collected by the RCM. As a result, the X-ray CT apparatus 1 can collect images for easier observation with less data generation and processing. In addition, the X-ray CT apparatus 1 can generate data having the high material discrimination performance in material discrimination.

According to the first embodiment, the second data has a spatial resolution that is approximately the same as the data acquired in the second mode (SEM), which performs coincidence counting correction. Thus, the X-ray CT apparatus 1 according to the first embodiment can restore data having a high spatial resolution, which is approximately the same as that of the data collected by the SEM.

According to the first embodiment, the model is trained based on the third data acquired in the first mode and the fourth data acquired in the second mode. The first mode is the mode of double counting data between adjacent anode pixels (RCM). The second mode is the mode of discarding double-counted data between adjacent anode pixels (SEM). Thus, the X-ray CT apparatus 1 according to the first embodiment can restore data by using the trained model generated from the data collected by the RCM and the data collected by the SEM.

According to the first embodiment, the third data and the fourth data are individually acquired under the scanning condition in which the counts are approximately the same as each other. The fourth data is acquired under the condition in which the amount of rays per unit time is higher than that of the third data. The fourth data is acquired under the condition in which the scan speed is slower than that of the third data. Therefore, the X-ray CT apparatus 1 according to the first embodiment can restore the data having a high spatial resolution, which is approximately the same as that of the data collected by the SEM, based on the data collected by the RCM.

### Other Embodiments

The first embodiment has been described so far, but the present embodiment may be implemented in various different forms other than the first embodiment described above.

It has been described in the above embodiment that the data is restored by using the trained model. However, the embodiment is not limited thereto, and data may be restored by using analytical models that analyze the mutual relationship between the data collected by the RCM and the data collected by the SEM, or by using a function.

In the embodiment described above, the case in which the X-ray CT apparatus 1 acquires the training data has been described. However, the embodiment is not limited thereto, and for example, data acquired by an X-ray CT apparatus different from the X-ray CT apparatus 1 may be used as the training data, as long as the geometric arrangement in a gantry and a couch device and a response in an X-ray detector are equivalent to those in the X-ray CT apparatus 1.

In the embodiment described above, the case in which the data of SEM is collected by the control of the ASIC and used as the training data has been described. However, the embodiment is not limited thereto, and for example, a shielding material may be disposed at the boundary between adjacent pixels (the boundary between the adjacent anode electrodes) to collect data, which is the same as that in the SEM, to be used as the training data. Thus, for example, the above shielding material can be used to collect training data even when the ASIC in the X-ray detector cannot perform SEM.

In the embodiment described above, the processing circuitry 44 is described to perform a plurality of the functions, but the embodiment is not limited thereto. For example, the functions may be provided in the console 40 as independent circuits, and each circuit may execute each function.

Each of the components of each device illustrated in the above-described embodiment is a functional concept, and does not necessarily have the physical configuration as illustrated in the figures. That is, the specific form of dispersion and integration of each device is not limited to those illustrated in the figures, and all or part thereof can be functionally or physically distributed and integrated in any units according to various loads and usage conditions. Furthermore, each processing function performed by each device can be implemented, in whole or in part, by CPU and a computer program analyzed and executed by the CPU, or by hardware using wired logic.

In addition, some of the processes described in the above embodiment as being performed automatically can be performed manually, or all or some of the processes described as being performed manually can be performed automatically by known methods. Other than those, information including processing procedures, control procedures, specific names, and various types of data and parameters illustrated in the above documents and figures may be changed as desired, unless otherwise specified.

The term "processor" used in the above description means a circuit such as a central processing unit (CPU), a graphics processing unit (GPU), an application specific integrated circuit (ASIC), or a programmable logic device (for example, simple programmable logic device (SPLD), complex programmable logic device (CPLD), and field programmable gate array (FPGA)), for example. The processor reads and executes the computer programs stored in the memory 41 to execute the functions. Instead of storing the computer programs in the memory 41, the computer programs may be configured to be incorporated directly into the circuit of the processor. In this case, the processor reads and executes the computer programs incorporated in the circuit to execute the functions thereof. Each processor of the present embodiment is not limited to the configuration of a single circuit for each processor, but may also employ the configuration of a single processor formed of a plurality of independent circuits in combination to execute the functions thereof. Furthermore, a plurality of the components in each figure may be integrated into a single processor to execute the functions thereof.

Here, the data processing method described in the above embodiment can be achieved by executing a pre-prepared data processing program by a computer such as a personal computer or workstation. This data processing program is provided incorporated in advance in a read only memory (ROM), a memory, or the like. The data processing program may be stored and provided in a format that can be installed in these devices or as a file in an executable format in a computer-readable storage medium, such as a compact disc (CD)-ROM, flexible Disk (FD), CD-recordable (R), digital versatile disc (DVD), or other storage medium. The data processing program may also be stored in a computer connected to a network, such as the Internet, and provided or distributed by downloading via the network. For example, this data processing program constitutes modules that include each of the functional units described below. As for the actual hardware, the CPU reads and executes the computer program from a storage medium such as a ROM, and each module is loaded in the main storage device and generated in the main storage device.

According to at least one of the embodiments described above, it is possible to collect images for easier observation in the image collection by using the photon-counting type X-ray detector.

While certain embodiments have been described, these embodiments have been presented by way of example only, and are not intended to limit the scope of the inventions. Indeed, the novel embodiments described herein may be embodied in a variety of other forms; furthermore, various omissions, substitutions and changes in the form of the embodiments described herein may be made without departing from the scope of the invention as defined by the appended claims.

## Claims

1. An X-ray CT apparatus (1) comprising:
an acquisition unit (441) configured to acquire first data in a first mode without coincidence counting correction; and
a restoration unit (445) configured to restore, from the first data, second data having a higher spatial resolution than the first data.

2. The X-ray CT apparatus (1) according to claim 1, wherein the restoration unit (445) restores the second data by inputting the first data to a trained model.

3. The X-ray CT apparatus (1) according to claim 1, wherein the restoration unit (445) restores the second data from the first data by using an analytical model that analyzes a mutual relationship between the first data and the second data, or a function.

4. The X-ray CT apparatus (1) according to any one of claims 1 to 3, wherein the second data has approximately the same spatial resolution as data acquired in a second mode with the coincidence counting correction.

5. The X-ray CT apparatus (1) according to claim 2, wherein the model is trained based on third data acquired in the first mode and fourth data acquired in the second mode.

6. The X-ray CT apparatus (1) according to claim 5, wherein the third data and the fourth data are individually acquired under a scanning condition in which counts are approximately the same as each other.

7. The X-ray CT apparatus (1) according to claim 6, wherein the fourth data is acquired under a condition in which the amount of rays per unit time is higher than the third data.

8. The X-ray CT apparatus (1) according to claim 6, wherein the fourth data is acquired under a condition in which a scan speed is slower than the third data.

9. The X-ray CT apparatus (1) according to any one of claims 1 to 8, wherein the first mode is a mode of double counting data between adjacent anode pixels.

10. The X-ray CT apparatus (1) according to claim 4, wherein the second mode is a mode of discarding double-counted data between adjacent anode pixels.

11. The X-ray CT apparatus (1) according to any one of claims 1 to 10, wherein the first data and the second data are projection data, CT image data obtained by performing reconstruction processing on projection data, or image data obtained by performing image processing on CT image data.

12. A data processing method comprising:
acquiring first data in a first mode without coincidence counting correction; and
restoring, from the first data, second data having a higher spatial resolution than the first data.

13. A computer program configured to cause a computer to execute each of processes of:
acquiring first data in a first mode without coincidence counting correction; and
restoring, from the first data, second data having a higher spatial resolution than the first data.
